# EUROPÄISCHE PATENTSCHRIFT

(11) **EP 2 929 329 B1**
(45) Veröffentlichungstag und Bekanntmachung des Hinweises auf die Patenterteilung: **06.05.2020**
(21) Anmeldenummer: 13811342.8
(22) Anmeldetag: 02.12.2013
(51) Int. Cl.: G01N 21/89

(54) **VORRICHTUNG ZUR OPTISCHEN PRÜFUNG EINES BEWEGTEN TEXTILMATERIALS**
DEVICE FOR THE OPTICAL INSPECTION OF A MOVING TEXTILE MATERIAL
DISPOSITIF POUR LE CONTRÔLE OPTIQUE D'UN TEXTILE EN MOUVEMENT

(30) Priorität: 10.12.2012 CH 27392012
(43) Veröffentlichungstag der Anmeldung: 14.10.2015
(73) Patentinhaber: Uster Technologies AG, 8610 Uster (CH)
(72) Erfinder: STORZ, Rafael, CH-8280 Kreuzlingen (CH); PIRANI . Peter, CH-8624 Grüt (CH)
(74) Vertreter: Pliska, Pavel
(86) Internationale Anmeldenummer: PCT/CH2013/000209
(87) Internationale Veröffentlichungsnummer: WO 2014/089713

(56) Entgegenhaltungen:
- EP-A1- 0 898 163
- WO-A1-01/99248
- WO-A1-2010/000078
- DE-A1- 3 830 665
- US-A- 5 854 683
- US-B1- 6 219 136

## Beschreibung

### FACHGEBIET

Die vorliegende Erfindung liegt auf dem Gebiet der textilen Materialprüfung. Sie betrifft eine Vorrichtung und ein Verfahren zur optischen Prüfung eines bewegten Textilmaterials, gemäss den Oberbegriffen der unabhängigen Patentansprüche. Die Erfindung kann bspw. in Gamprüfgeräten im Textillabor oder in Garnreinigern auf Spinn- oder Spulmaschinen eingesetzt werden.

### STAND DER TECHNIK

Es ist eine Vielzahl verschiedenartiger Vorrichtungen zur Prüfung von Textilmaterialien bekannt. In den Textilprüfvorrichtungen kommen verschiedene Sensorprinzipien zur Anwendung; der Einsatz eines bestimmten Sensorprinzips hängt unter anderem davon ab, welche Eigenschaft optimal detektiert werden soll. Häufig verwendete Sensorprinzipien, besonders in der Gamprüfung, sind die folgenden:
- Das kapazitive Sensorprinzip; vgl. US-6,346,819 B1. Das Textilmaterial wird durch einen Luftspalt eines Messkondensators geleitet. Der Messkondensator misst im Wesentlichen die Masse des in ihm befindlichen Textilmaterials. Das kapazitive Sensorprinzip weist eine hohe Messgenauigkeit und eine über lange Zeit stabile Empfindlichkeit auf. Seine Nachteile sind eine unerwünschte Empfindlichkeit gegenüber Feuchtigkeitsänderungen und die Nichtverwendbarkeit mit elektrisch leitenden Textilmaterialien.
- Das optische Sensorprinzip; vgl. WO-2004/044579 A1. Das Textilmaterial wird von einer Lichtquelle beleuchtet, und mit dem Textilmaterial wechselwirkendes Licht wird von Lichtdetektoren detektiert. Das detektierte Licht ist ein Mass für den Durchmesser des Textilmaterials und/oder seine optischen Eigenschaften wie Reflektivität oder Farbe. Das optische Sensorprinzip ist weniger empfindlich gegenüber Durchmesseränderungen und langfristig weniger stabil als das kapazitive. Trotzdem kann es vorteilhaft sein, besonders für solche Anwendungen, für welche das kapazitive Sensorprinzip ungeeignet ist, bspw. in Umgebungen mit starken Feuchtigkeitsschwankungen oder für elektrisch leitende Textilmaterialien. Fremdstoffe, die eine vom Textilmaterial stark abweichende Reflektivität haben, können mit dem optischen Sensorprinzip auf einfache Weise detektiert werden.

Es wurde schon vorgeschlagen, Garn mit unterschiedlichen Sensorprinzipien abzutasten und die Sensorsignale bei der Auswertung miteinander zu verknüpfen. So erwähnt die CN-2'896'282 Y die Kombination eines kapazitiven und eines photoelektrischen Sensors zur Detektion der Massendichte und des Durchmessers desselben Garns. Die WO-01/92875 A1 lehrt, zwei Sensoren hintereinander entlang des Garnpfades anzuordnen. Ein erster der Sensoren misst die optische Reflexion am Garn; ein zweiter der Sensoren misst kapazitiv oder optisch die Masse bzw. den Durchmesser des Garns. Die Ausganssignale der beiden Sensoren werden gemäss bestimmten Auswertekriterien ausgewertet. Aufgrund der Auswertung können mindestens zwei Arten von Fremdstoffen voneinander unterschieden werden.

Die WO-93/13407 A1 gibt ein Beispiel für einen optischen Gamreinigermesskopf zur Detektion von Fremdfasern an. Das durch einen Messspalt bewegte Garn wird von einer Lichtquelle mit moduliertem Licht beleuchtet. Ein erster Sensor nimmt vom Garn reflektiertes Licht und gleichzeitig ein zweiter Sensor vom Garn transmittiertes Licht auf. Aus den von den beiden Sensoren ausgegebenen elektrischen Signalen wird auf die Anwesenheit einer Fremdfaser im Garn geschlossen. Zur Lichtleitung zwischen der Lichtquelle bzw. den Sensoren und dem Messspalt sind dreidimensionale Lichtzuführer vorgesehen, die z. B. als innen verspiegelte Hohlräume ausgebildet sind.

Die US-5,768,938 A reduziert gegenüber der WO-93/13407 A1 den Platzbedarf des Messkopfes, indem die Lichtzuführer in einer Ebene angeordnet sind, die senkrecht zum Garn steht. Die Lichtzuführer sind als ein dreidimensionaler, Licht übertragender Körper ausgebildet, der in einen dreidimensionalen Grundkörper eingesetzt ist. Der Grundkörper weist auch eine Aufnahmeöffnung für eine Lichtquelle auf. Selbst diese Vorrichtung braucht noch relativ viel Platz. Wenn eine Änderung des optischen Abtastteils nötig ist, müssen der ganze Messkopf neu entworfen und die entsprechenden Herstellungswerkzeuge neu gebaut werden, was äusserst aufwändig ist.

Die DE-38'30'665 A1 offenbart eine optoelektronische Vorrichtung zur Fadenüberwachung. Alle aktiven optoelektronischen Bauelemente wie Leuchtdioden und Fototransistoren sind in einer Zentraleinheit angebracht. Die Zentraleinheit ist mittels Lichtleiter mit mehreren Fadenwächtern verbunden, die sich jeweils an einer Fadenlaufstelle befinden. Ein Fadenwächter besteht nur aus einer Platine mit einer Fadenführungsöse. Die Enden zweier mit der Zentraleinheit verbundener Lichtleiter sind derart in je eine Öffnung in der Platine eingesteckt, dass sie einander gegenüber liegen.

Aus der DE-10'2007'040'224 A1 ist ein optoelektronischer Garnsensor bekannt. Die Bauelemente des Garnsensors sind auf einer Platine angeordnet. Eine Sendediode emittiert Licht, das von einer Linse auf das Garn gelenkt wird. Das vom Garn transmittierte Licht wird von einer Empfangsdiode detektiert. Ein Teil des von der Sendediode emittierten Lichtes wird von der Linse abgespalten und über einen Lichtleiter, der in die Linse integriert ist, einer Monitordiode zugeführt. Der Strom der Sendediode wird in Abhängigkeit vom Signal der Monitordiode geregelt, so dass sich eine konstante Sendelichtstärke ergibt.

Die WO-2010/000078 A1 offenbart eine Vorrichtung zur Erfassung von Parametern an einem fadenförmigen Prüfgut. Eine erste und eine zweite Messzelle sind auf einem gemeinsamen ebenen Träger angeordnet. Die erste Messzelle kann optisch, die zweite Messzelle kann kapazitiv arbeiten.

Die EP-0'898'163 A1 offenbart eine Vorrichtung für die automatische Inspektion sich bewegender Oberflächen. Gemäss einer Ausführungsform wird für die Beleuchtung der Oberfläche eine Faseroptikeinrichtung verwendet. Diese beinhaltet drei Lichtleiterschichten, die jeweils aus einer Vielzahl von seitlich aneinander gereihten Lichtleiterfasern bestehen. Die drei Lichtleiterschichten sind zwischen zwei Deckschichten eingebracht.

Leiterplatten mit integrierten optischen Wellenleiterstrukturen und integrierten elektrischen Leiterstrukturen sind an sich bekannt, bspw. aus der US-2010/0209854 A1.

### DARSTELLUNG DER ERFINDUNG

Es ist eine Aufgabe der vorliegenden Erfindung, eine Vorrichtung zur optischen Abtastung eines bewegten Textilmaterials anzugeben, welche noch weniger Platz beansprucht als die aus dem Stand der Technik bekannten Vorrichtungen. Ausserdem soll die Vorrichtung vielseitig einsetzbar sein und mit wenig Aufwand verändert oder gewartet werden können.

Diese und andere Aufgaben werden durch die erfindungsgemässe Vorrichtung, wie sie im ersten Patentanspruch definiert ist, gelöst. Vorteilhafte Ausführungsformen sind in den abhängigen Patentansprüchen angegeben.

Gemäss der Erfindung wird ein Substrat mit mindestens einer darauf integrierten optischen Wellenleiterstruktur zur optischen Prüfung eines bewegten, vorzugsweise länglichen Textilmaterials verwendet. Auf dem Substrat kann zusätzlich eine elektrische Leiterstruktur zur elektrischen Prüfung des bewegten Textilmaterials integriert sein.

Unter einer auf einem Substrat integrierten optischen Wellenleiterstruktur wird in dieser Schrift eine monolithisch in oder auf dem Substrat untergebrachte Wellenleiterstruktur verstanden. Die Wellenleiterstruktur wurde originär auf dem Substrat hergestellt, bspw. durch Technologien wie Fotolithografie und/oder Dotierung - im Gegensatz zu eigenständigen, diskreten Wellenleitern, die nachträglich auf ein Substrat aufgebracht wurden. Die integrierte optische Wellenleiterstruktur ist untrennbar mit dem Substrat verbunden. Sie beinhaltet eine Mehrzahl von transparenten dielektrischen Schichten mit unterschiedlichen Brechzahlen. Vorzugsweise ist eine Kernschicht mit höherer Brechzahl zwischen einer Unter- und einer Oberschicht mit niedrigeren Brechzahlen eingebettet, so dass Lichtwellen in der Kernschicht geleitet werden können. Die Wellenleiterstruktur kann Streifenwellenleiter, die Licht in einer Richtung leiten, und/oder flächige Dünnschichtleiter, in denen sich Licht in zwei Richtungen ausbreiten kann, beinhalten. Nebst den Wellenleitern kann sie weitere passive und/oder aktive integriert-optische Bauteile wie Linsen, Strahlteiler, Reflektoren, Filter, Verstärker, Lichtquellen und/oder Lichtempfänger beinhalten.

Die erfindungsgemässe Vorrichtung zur optischen Prüfung eines bewegten, vorzugsweise länglichen Textilmaterials beinhaltet also ein Substrat, auf dem ein Abtastbereich zum optischen Abtasten des Textilmaterials vorgesehen ist, und eine auf dem Substrat integrierte optische Wellenleiterstruktur, die zumindest teilweise in den Abtastbereich mündet.

In einer bevorzugten Ausführungsform münden mindestens zwei optische Wellenleiter der optischen Wellenleiterstruktur in den Abtastbereich. Dabei können mindestens zwei Wellenleiter zur Leitung von Licht zum Abtastbereich hin und mindestens zwei Wellenleiter zur Leitung von Licht vom Abtastbereich weg ausgebildet sein. Die Mündungen der hinleitenden bzw. der wegleitenden Wellenleiter sind vorzugsweise abwechslungsweise nebeneinander angeordnet.

Zur Vermeidung von Lichtverlusten ist es von Vorteil, wenn mindestens eine Mündung eines Wellenleiters in den Abtastbereich mit einer Sammellinse versehen ist.

Die optische Wellenleiterstruktur kann mindestens eine Verzweigung mit mindestens zwei Ästen aufweisen. Dabei können mindestens zwei der Äste dem Abtastbereich zugewandt oder vom Abtastbereich abgewandt sein.

In einer weiteren bevorzugten Ausführungsform weist das Substrat ausserhalb des Abtastbereichs mindestens eine optische Schnittstelle zum Verbinden der mindestens einen optischen Wellenleiterstruktur mit jeweils einem optischen Anschlussteil auf. Die mindestens eine Schnittstelle weist vorzugsweise mechanische Positioniermittel zum Positionieren des optischen Anschlussteils bezüglich des Substrates auf. Es kann eine Einkoppelschnittstelle zum Einkoppeln von Licht in mindestens eine optische Wellenleiterstruktur und eine von der Einkoppelschnittstelle verschiedene Auskoppelschnittstelle zum Auskoppeln von Licht aus mindestens einer optischen Wellenleiterstruktur vorhanden sein. Die mindestens eine optische Schnittstelle ist vorzugsweise zum Verbinden von mindestens zwei optischen Wellenleitern mit einem optischen Anschlussteil ausgebildet. Sie ist bevorzugt an einem Rand des Substrates angebracht.

Die Erfindung betrifft auch eine Kombination einer erfindungsgemässen Vorrichtung, welche die oben beschriebene mindestens eine optische Schnittstelle aufweist, mit einem optischen Anschlussteil. Ein der Einkoppelschnittstelle zugeordnetes Einkoppelanschlussteil kann dabei eine Reihe von mindestens zwei Lichtquellen, vorzugsweise ein Leuchtdiodenarray, und ein der Auskoppelschnittstelle zugeordnetes Auskoppelanschlussteil kann eine Reihe von mindestens zwei Lichtempfängern, vorzugsweise ein CCD-Array, beinhalten.

Gemäss einer weiteren bevorzugten Ausführungsform ist auf dem Substrat zusätzlich eine elektrische Leiterstruktur integriert, die zumindest teilweise in den Abtastbereich mündet. Damit kann das Textilmaterial wahlweise optisch, elektrisch oder sowohl optisch als auch elektrisch untersucht werden. Vorteilhafterweise münden mindestens zwei elektrische Leiterbahnen der elektrischen Leiterstruktur in den Abtastbereich. Die Mündungen der optischen Wellenleiter und die Mündungen der elektrischen Leiterbahnen können abwechslungsweise nebeneinander angeordnet sein. Mindestens eine Mündung einer elektrischen Leiterbahn ist vorzugsweise im Abtastbereich mit einer Elektrode versehen. Das Substrat kann ausserhalb des Abtastbereichs mindestens eine elektrische Schnittstelle zum Verbinden der mindestens einen elektrischen Leiterstruktur mit jeweils einem elektrischen Anschlussteil aufweisen. Die mindestens eine elektrische Schnittstelle weist vorzugsweise mechanische Positioniermittel zum Positionieren des elektrischen Anschlussteils bezüglich des Substrates auf. Sie ist z. B. an einem Rand des Substrates angebracht.

Bei Vorrichtungen, die ein Substrat mit einer optischen Wellenleiterstruktur und einer elektrischen Leiterstruktur kann das Substrat ausserhalb des Abtastbereichs mindestens eine optisch-elektrische Schnittstelle zum Verbinden der mindestens einen optischen Wellenleiterstruktur und der mindestens einen elektrischen Leiterstruktur mit jeweils einem optisch-elektrischen Anschlussteil aufweisen. Die mindestens eine optisch-elektrische Schnittstelle weist vorzugsweise mechanische Positioniermittel zum Positionieren des optisch-elektrischen Anschlussteils bezüglich des Substrates auf.

Die Integration der optischen Wellenleiterstruktur auf dem Substrat führt zu einer Platzersparnis gegenüber bekannten optischen Vorrichtungen zur Prüfung von Textilmaterialien. Ausserdem kann in der erfindungsgemässen Vorrichtung das Substrat einfach ausgewechselt werden, um die Vorrichtung zu warten oder zu verändern. Eine Veränderung der Vorrichtung kann durch das Ersetzen einer bestimmten Substrates durch ein anderes Substrat erfolgen, in dem z. B. der Abtastbereich unterschiedlich gestaltet ist. Es kann bspw. ein erstes Substrat bereitgestellt werden, mittels dessen das Textilmaterial nur von einer Seite untersucht wird, und ein zweites Substrat, mittels dessen das Textilmaterial von mehreren Richtungen entlang seines Umfangs untersucht wird.

In der vorliegenden Schrift werden Begriffe wie "Licht" oder "beleuchten" nicht nur für sichtbares Licht, sondern auch für elektromagnetische Strahlung aus den angrenzenden Spektralbereichen Ultraviolett (UV) und Infrarot (IR) verwendet.

### AUFZÄHLUNG DER ZEICHNUNGEN

Nachfolgend wird die Erfindung anhand der schematischen Zeichnungen detailliert erläutert.
- Figuren 1-4: zeigen schematisch vier verschiedene Ausführungsformen einer erfindungsgemässen Vorrichtung in Aufsichten.
- Figur 5: zeigt schematisch ein Ende eines optischen Wellenleiters, ein zu prüfendes Textilmaterial und dazwischen verlaufende Lichtstrahlen in einer Aufsicht.
- Figuren 6-9: zeigen schematisch vier weitere Ausführungsform einer erfindungsgemässen Vorrichtung in Aufsichten.
- Figuren 10 und 11: zeigen schematisch zwei weitere Ausführungsformen einer erfindungsgemässen Vorrichtung in perspektivischen Ansichten.
- Figur 12: zeigt schematisch eine elfte Ausführungsform einer erfindungsgemässen Vorrichtung in einem Querschnitt.

### AUSFÜHRUNG DER ERFINDUNG

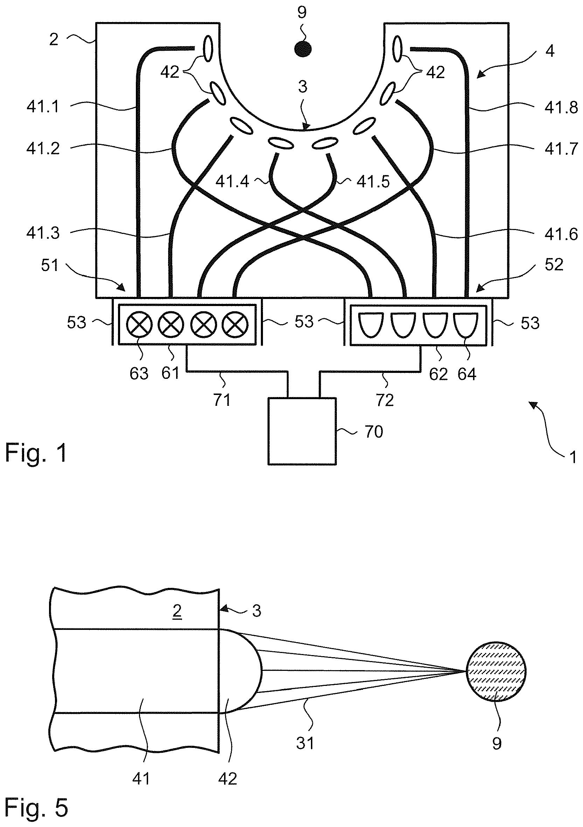

**Figur 1** zeigt eine erste Ausführungsform einer erfindungsgemässen Vorrichtung 1. Die Vorrichtung 1 dient der Prüfung eines vorzugsweise länglichen Textilmaterials 9, bspw. eines Garns, das durch die Vorrichtung 1 oder an der Vorrichtung 1 vorbei bewegt wird. Im vorliegenden Fall verläuft die Bewegungsrichtung des Textilmaterials 9 senkrecht zur Zeichenebene, in Richtung einer Längsachse des Textilmaterials 9. Die Vorrichtung 1 beinhaltet ein Substrat 2, auf dem ein Abtastbereich 3 zum optischen Abtasten des Textilmaterials 9 vorgesehen ist. Das Substrat 2 kann aus einem an sich bekannten Material wie Glas, einem Kunststoff, einem Halbleitermaterial oder einer mit Epoxidharz getränkten Glasfasermatte bestehen. Es ist vorzugsweise eben und rigid, d. h. verformt sich praktisch nicht.

Der Abtastbereich 3 ist im vorliegenden Ausführungsbeispiel als eine im Wesentlichen halbrunde Aussparung an einem Rand des Substrates 2 ausgeführt. Das Textilmaterial 9 wird so durch die Vorrichtung 1 geführt, dass seine Längsachse möglichst im Mittelpunkt des Halbkreises liegt. Die Ebene des Substrates 2 liegt senkrecht zur Längsachse des Textilmaterials 9.

Auf dem Substrat 2 ist eine optische Wellenleiterstruktur 4 zur Leitung von Licht zum Abtastbereich 3 hin und/oder vom Abtastbereich 3 weg integriert. Im Ausführungsbeispiel von Figur 1 beinhaltet die Wellenleiterstruktur 4 acht optische Streifenwellenleiter 41.1-41.8, die jeweils den Abtastbereich 3 mit einer optischen Schnittstelle 51, 52 verbinden. Die Wellenleiterstruktur 4 kann z. B. aus einem Polymer gefertigt sein, das für die verwendete Lichtwellenlänge genügend durchlässig ist. Sie wird vorzugsweise durch einen fotolithografischen Prozess auf das Substrat 2 aufgebracht. Die Querdimensionen (Breite und Höhe) eines einzelnen Wellenleiters 41.1-41.8 können z. B. zwischen 5 µm und 500 µm, vorzugsweise ca. 50 µm, betragen. Die Wellenleiterstruktur 4 kann sich auf einer äussersten Schicht des Substrates 2 befinden oder eine Innenschicht bilden, die durch mindestens eine darüber liegende Schicht bedeckt ist. Im letzteren Fall kann die darüber liegende Schicht die Wellenleiterstruktur 4 vor mechanischen Beschädigungen, Verschmutzung und unerwünschten optischen Einflüssen schützen. Die Wellenleiter 41.1-41.8 können als Singlemode- oder als Multimode-Wellenleiter ausgeführt sein. Die Wellenleiterstruktur 4 von Figur 1 weist mehrere Kreuzungen von Wellenleitern 41.1-41.8 auf. Es sollte darauf geachtet werden, dass an diesen Kreuzungen ein Übersprechen vom einen auf den anderen Wellenleiter vermieden wird. Der Fachmann auf dem Gebiet der integrierten Optik ist in der Lage, die Wellenleiterstruktur 4 so auszulegen, dass diese Bedingung gut erfüllt ist. Dies kann insbesondere dann der Fall sein, wenn der jeweilige Kreuzungswinkel nahe bei 90° liegt oder zumindest nicht zu spitz ist. Nebst den Wellenleitern 41.1-41.8 selbst kann die integrierte optische Wellenleiterstruktur 4 weitere integriert-optische Bauteile wie Linsen, Strahlteiler, Reflektoren, Filter, Verstärker, Lichtquellen und/oder Lichtempfänger beinhalten.

An den Mündungen der Wellenleiter 41.1-41.8 in den Abtastbereich 3 sind vorzugsweise Licht sammelnde Elemente 42 wie Sammellinsen angebracht, um eine möglichst hohe Lichtausbeute zu gewährleisten. Bekanntlich wird aus dem Ende eines dünnen Wellenleiters austretendes Licht in einem grossen Öffnungswinkel abgestrahlt; in zeitlicher Umkehr wird daher auch Licht aus demselben grossen Öffnungswinkel in den Wellenleiter eingekoppelt. Da das zu prüfende Textilmaterial 9 meist einen kleinen Durchmesser von weniger als 1 mm hat, würde es ohne Gegenmassnahmen von bloss einem kleinen Teil des zur Verfügung stehenden Lichtes getroffen, und von diesem würde wiederum nur bloss ein kleiner Teil wieder in einen Wellenleiter eingekoppelt. Die Sammellinsen 42 dienen dazu, solche Lichtverluste zu vermeiden. Ihre Funktion und ihr Aufbau werden weiter unten anhand der Figur 5 detaillierter erklärt.

Vier der acht Wellenleiter 41.1-41.8, die nachfolgend als "Beleuchtungswellenleiter" 41.1, 41.3, 41.5, 41.7 bezeichnet werden, dienen der Beleuchtung des Textilmaterials 9. Zu diesem Zweck nehmen sie Licht von einem Sendermodul 61 auf und leiten es in den Abtastbereich 3, wo es aus den Beleuchtungswellenleitern 41.1, 41.3, 41.5, 41.7 austritt und zumindest teilweise auf das Textilmaterial 9 trifft. Das Sendermodul 61 kann am Ende einer ersten elektrischen Leitung 71 angebracht sein. Die Lichtübergabe vom Sendermodul 61 in die Beleuchtungswellenleiter 41.1, 41.3, 41.5, 41.7 erfolgt an einer Einkoppelschnittstelle 51, die an einem Rand des Substrates 2 angebracht ist. Die Einkoppelschnittstelle 51 kann z. B. als Steckverbindung ausgestaltet sein. Das Sendermodul 61 beinhaltet bspw. in einer Reihe nebeneinander angeordnete Lichtquellen 63, von denen jede einem der vier Beleuchtungswellenleiter 41.1, 41.3, 41.5, 41.7 zugeordnet ist. Die Lichtquellen 63 können z. B. als Diodenlaser oder als Leuchtdioden ausgeführt sein. Die Einkopplung des Lichtes von den Lichtquellen 63 in die Beleuchtungswellenleiter 41.1, 41.3, 41.5, 41.7 kann durch direkte Beleuchtung der Beleuchtungswellenleiterenden oder mittels optischer Elemente wie Spiegel und/oder Sammellinsen 42 erfolgen. Im letzteren Fall können ähnliche Linsen eingesetzt werden wie an der Mündung zum Abtastbereich 3; siehe Figur 5. Zur Gewährleistung einer wirksamen Einkopplung des Lichtes in die Beleuchtungswellenleiter 41.1, 41.3, 41.5, 41.7 an der Einkoppelschnittstelle 51 ist es wichtig, dass die Lichtquellen 63 bezüglich der Beleuchtungswellenleiterenden möglichst exakt und stabil positioniert werden. Zu diesem Zweck sind vorzugsweise an der Einkoppelschnittstelle 51 mechanische Positioniermittel 53 zum Positionieren des Sendermoduls 61 bezüglich des Substrates 2 angebracht. Die Positioniermittel 53 können z. B. als geeignete Führungen ausgebildet sein, die für eine genaue Positionierung innerhalb der Steckverbindung sorgen. Sie sind, wie auch die anderen Elemente, in Figur 1 bloss schematisch angedeutet.

Die übrigen vier der acht Wellenleiter 41.1-41.8 dienen der Detektion des vom Textilmaterial 9 reflektierten oder an diesem vorbei transmittierten Lichtes und werden deshalb nachfolgend als "Detektionswellenleiter" 41.2, 41.4, 41.6, 41.8 bezeichnet. Sie leiten das Licht, das vom Abtastbereich 3 kommt, zu einem Empfängermodul 62, das am Ende einer zweiten elektrischen Leitung 72 angebracht sein kann. Die Lichtübergabe von den Detektionswellenleitem 41.2, 41.4, 41.6, 41.8 in das Empfängermodul 62 erfolgt an einer Auskoppelschnittstelle 52, die an einem Rand des Substrates 2 angebracht ist. Die Auskoppelschnittstelle 52 kann ebenfalls als Steckverbindung mit entsprechenden Positioniermitteln 53 ausgestaltet sein. Das Empfängermodul 62 beinhaltet bspw. in einer Reihe nebeneinander angeordnete Lichtempfänger 64, von denen jeder einem der vier Detektionswellenleiter 41.2, 41.4, 41.6, 41.8 zugeordnet ist. Die Lichtempfängerreihe kann z. B. als CCD-Array ausgeführt sein. Es könnten auch mehrere nebeneinander liegende Empfangselemente, die dann einen "zusammengesetzten Lichtempfänger" bilden, zusammengefasst und einem Detektionswellenleiter 41.2, 41.4, 41.6, 41.8 zugeordnet werden. Bezüglich der Lichtauskopplung sowie der Positionierung und Gestaltung der Auskoppelschnittstelle 52 gilt analog das zur Einkoppelschnittstelle 51 Gesagte.

Das Sendermodul 61 und das Empfängermodul 62 sind über die erste elektrische Leitung 71 bzw. die zweite elektrische Leitung 72 mit einer Elektronikeinheit 70 verbunden. Diese steuert einerseits das Sendermodul 61 an. Andererseits empfängt die Elektronikeinheit 70 Signale des Empfängermoduls 62, wertet sie selbst aus oder leitet sie, möglicherweise nach einer Vorverarbeitung, an eine (nicht eingezeichnete) Auswerteeinheit weiter.

Es ist vorteilhaft, die Einkoppelschnittstelle 51 und die Auskoppelschnittstelle 52 optisch und mechanisch genau zu definieren und so quasi zu standardisieren. Dann können das Sendermodul 61 und das Empfängermodul 62 mit ihren relativ teuren optoelektronischen Bauteilen ohne Änderungen für verschiedene Substrate 2 verwendet werden. Hingegen können die relativ kostengünstigen Substrate 2 mit ihren integrierten optischen Wellenleiterstrukturen 4 nach Bedarf ausgewechselt werden. Ein Bedarf nach Auswechslung eines Substrates 2 kann z. B. dann entstehen, wenn eine andere Wellenleiterstruktur 4 - besonders beim Abtastbereich 3 - gebraucht wird oder wenn ein Substrat 2 durch Verschleiss beschädigt oder anderswie defekt ist.

Die erfindungsgemässe Vorrichtung 1 ist vorzugsweise in einem Gehäuse untergebracht, wie es bspw. aus der US- 5,768,938 A bekannt ist. Der Übersichtlichkeit halber wurde ein solches Gehäuse in den beiliegenden Zeichnungen nicht eingezeichnet.

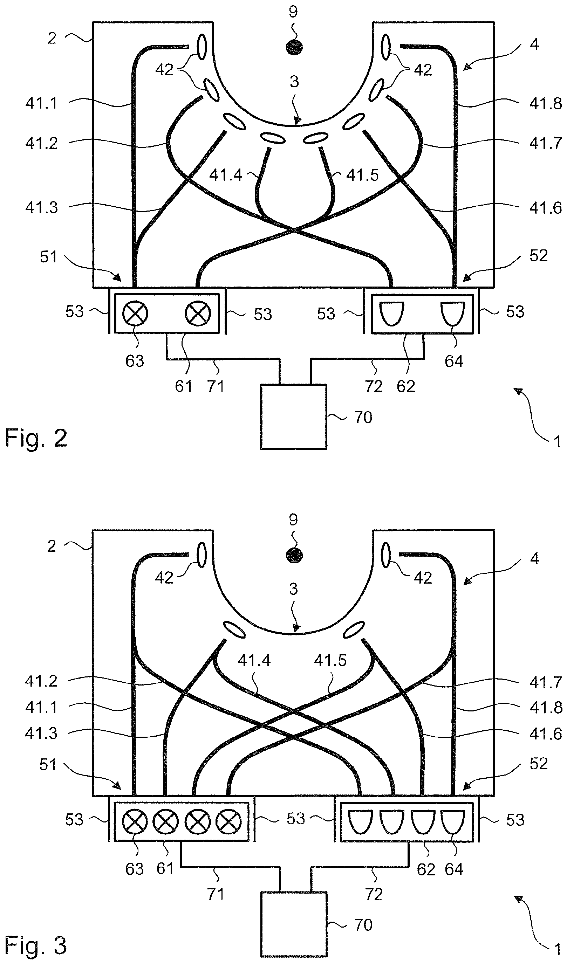

**Figur 2** zeigt eine zweite Ausführungsform der erfindungsgemässen Vorrichtung 1. Sie ist gegenüber der ersten Ausführungsform von Figur 1 in dem Sinn vereinfacht, als dass das Sendermodul 61 nur zwei Lichtquellen 63 und das Empfängermodul 62 nur zwei Lichtempfänger 64 aufweisen. Dabei ist die Anzahl der in den Abtastbereich 3 mündenden Wellenleiterenden dieselbe wie in der ersten Ausführungsform. Dies ist durch den Einsatz von Verzweigungen in der Wellenleiterstruktur 4 möglich. Jeder der vier dem Sendermodul 61 bzw. dem Empfängermodul 62 zugewandten Wellenleiter weist eine Y-Verzweigung auf, deren beiden Äste jeweils dem Abtastbereich 3 zugewandt sind, so dass sich beim Abtastbereich 3 doppelt so viele Wellenleiterenden befinden wie bei den Modulen 61, 62. Durch den Einsatz weiterer Verzweigungen könnte die Vorrichtung 1 sogar mit bloss einer Lichtquelle und/oder einem Lichtempfänger auskommen. Die erwähnte Vereinfachung der Module 61, 62 wird durch eine geringere Ortsauflösung erkauft. Die Signale je zweier Detektionswellenleiter 41.2, 41.4; 41.6, 41.8 werden nämlich zu einem einzigen Signal zusammengefasst und können nur gemeinsam detektiert werden. Dies muss aber nicht nachteilig sein. Wenn es z. B. darum geht, Fremdfasern im Textilmaterial 9 zu finden, interessiert ohnehin nur die Gesamtreflektivität des Textilmaterials 9 entlang seines Umfangs oder eines Teils davon. Für eine solche Anwendung ist also die zweite Ausführungsform genauso geeignet wie die erste.

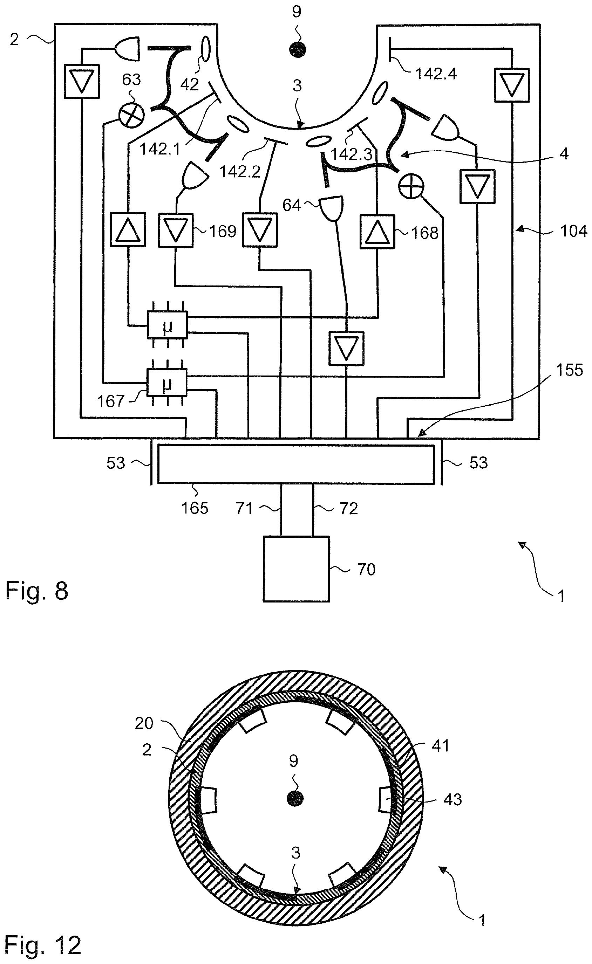

Die Verzweigungen können als an sich bekannte Verzweigerbauteile ausgeführt sein. Es kann sich, wie im Beispiel von Figur 2, um 1×2-Verzweigungen oder um Verzweigungen höherer Ordnung handeln. Nebst den Verzweigungen kann die optische Wellenleiterstruktur 4 weitere integriert-optische Bauteile beinhalten. Diese können passiv und/oder aktiv sein. Beispiele für solche integriert-optischen Bauteile sind Linsen, Strahlteiler, Reflektoren, Filter, Verstärker, Lichtquellen und Lichtempfänger. Ausserdem können optische Elemente wie Lichtquellen und/oder Lichtempfänger als eigenständige, diskrete Bauteile auf das Substrat 2 aufgebracht werden; siehe dazu Figur 8.

Eine dritte Ausführungsform der erfindungsgemässen Vorrichtung 1, die ebenfalls Y-Verzweigungen enthält, ist in **Figur 3** dargestellt. Hier besteht die Vereinfachung gegenüber der ersten Ausführungsform von Figur 1 in einer Halbierung der Anzahl der dem Abtastbereich 3 zugewandten Wellenleiterenden, ohne dass die Ortsauflösung reduziert würde. Dies wird erreicht, indem jedes dem Abtastbereich 3 zugewandte Wellenleiterende sowohl der Beleuchtung als auch der Detektion dient. Eine solche Mehrfachverwendung eines Wellenleiterabschnittes ist möglich, weil das Beleuchtungslicht und das Detektionslicht im selben Wellenleiterabschnitt einander nicht beeinflussen. Die Verzweigungen sind so ausgestaltet, dass ein grosser Teil des von der zugeordneten Lichtquelle 63 ausgesandten Lichtes in den Abtastbereich 3 abgestrahlt wird, und dass ein grosser Teil des vom Abtastbereich 3 empfangenen Lichtes dem zugeordneten Lichtempfänger 64 zugeführt wird. Der Fachmann auf dem Gebiet der integrierten Optik ist in der Lage, bei Kenntnis der Erfindung derartige Verzweigungen herzustellen.

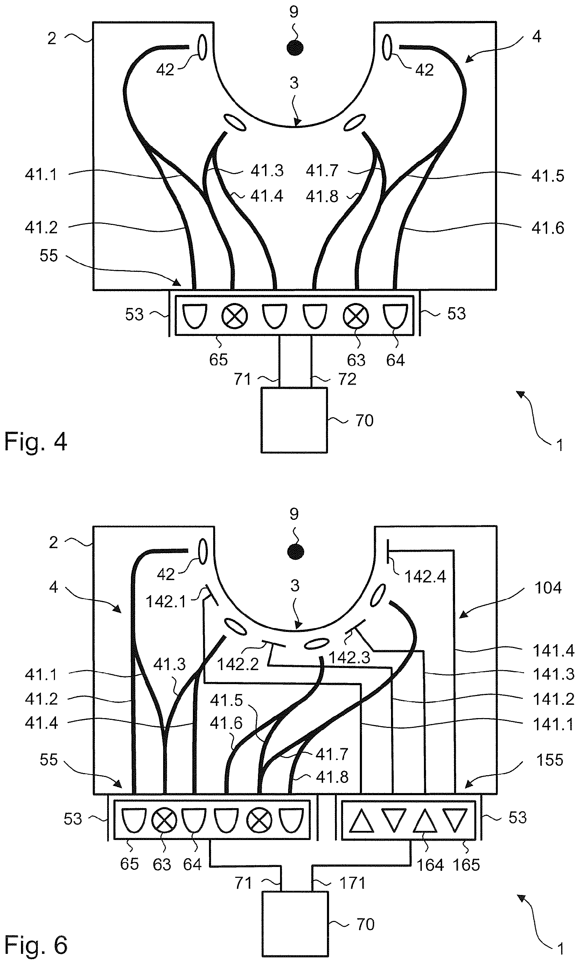

**Figur 4** zeigt eine vierte Ausführungsform der erfindungsgemässen Vorrichtung 1. Hier werden Y-Verzweigungen eingesetzt, um einerseits die Anzahl benötigter Lichtquellen 63 und andrerseits die Anzahl der dem Abtastbereich 3 zugewandten Wellenleiterenden gegenüber der ersten Ausführungsform von Figur 1 zu halbieren, ohne an Ortsauflösung zu verlieren. Ein weiterer Vorteil dieser Ausführungsform ist das Fehlen von Wellenleiterkreuzungen. Es ist nur eine einzige optische Schnittstelle 55 vorhanden, welche sowohl als Einkoppel- als auch als Auskoppelschnittstelle dient. Im entsprechenden Sender- und Empfängermodul 65 befinden sich somit sowohl Lichtquellen 63 als auch Lichtempfänger 64.

Ein dem Abtastbereich 3 zugewandtes Ende eines auf einem Substrat 2 integrierten Wellenleiters 41 ist schematisch in **Figur 5** stark vergrössert dargestellt. Dabei spielt es keine Rolle, ob es sich um einen Beleuchtungswellenleiter oder einen Detektionswellenleiter handelt, denn die beiden Fälle gehen durch Zeitumkehr ineinander über. Das Wellenleiterende ist mit einer Sammellinse 42 versehen. Die Sammellinse 42 kann aus dem Wellenleiterende selbst gefertigt, an dasselbe direkt angeklebt oder von diesem beabstandet sein. Sie ist derart beschaffen und angeordnet, dass sie möglichst viel aus dem Wellenleiter 41 austretendes Licht 31 auf das Textilmaterial 9 auftreffen lässt bzw. möglichst viel vom Textilmaterial 9 kommendes Licht 31 in den Wellenleiter 41 einkoppelt. Was in Figur 5 schematisch als eine einzige Sammellinse 42 eingezeichnet ist, kann in der Praxis als ein Linsensystem ausgeführt sein. Der Fachmann auf dem Gebiet der technischen Optik ist bei Kenntnis der Erfindung in der Lage, eine zum beschriebenen Zweck geeignete Anordnung zu bestimmen und einzusetzen.

**Figur 6** zeigt eine fünfte Ausführungsform der erfindungsgemässen Vorrichtung 1. Hier sind sowohl eine optische Wellenleiterstruktur 4 als auch eine elektrische Leiterstruktur 104 auf demselben Substrat 2 integriert, wobei beide Strukturen 4, 104 zumindest teilweise in den Abtastbereich 3 münden. Somit bietet diese Ausführungsform die Möglichkeit, das Textilmaterial 9 wahlweise optisch, elektrisch oder sowohl optisch als auch elektrisch zu prüfen.

Die optische Wellenleiterstruktur 4 und die optische Schnittstelle 51 in der Ausführungsform von Figur 6 entsprechen denjenigen der Ausführungsform von Figur 4. Der Einfachheit halber ist nur noch eine Verbindungsleitung 71 zwischen dem optischen Sender- und Empfängermodul 65 und der Elektronikeinheit 70 eingezeichnet, wobei jedoch mehrere Verbindungsleitungen vorhanden sein können.

Die elektrische Leiterstruktur 104 ist in Figur 6 sehr einfach ausgeführt. Sie beinhaltet vier elektrisch voneinander isolierte elektrische Leiterbahnen 141.1-141.4, welche jeweils vom Abtastbereich 3 zu einer elektrischen Schnittstelle 155 führen. Im Allgemeinen können auf dem Substrat 2 elektrische Schaltkreise integriert sein, wie sie aus dem Fachgebiet der Elektronik bekannt sind. Solche Schaltkreise können nebst elektrischen Leiterbahnen passive und/oder aktive elektrische Bauteile beinhalten. Beispiele für solche elektrischen Bauteile sind Widerstände, Kondensatoren, Spulen, Transistoren, Filter und Verstärker. Auch komplexe Bauteile wie Mikroprozessoren können sich auf dem Substrat 2 befinden, wobei diese vorzugsweise als integrierte Schaltkreise in einem eigenen Gehäuse auf das Substrat 2 aufgebracht werden; vgl. dazu Figur 8.

Die Mündungen der elektrischen Leiterbahnen 141.1-141.4 in den Abtastbereich 3 sind mit Elektroden 142 versehen. Die Elektroden 142 dienen dazu, ein elektrisches Feld, vorzugsweise ein elektrisches Wechselfeld, im Abtastbereich 3 zu erzeugen und/oder zu detektieren. Das Textilmaterial 9 wechselwirkt mit dem elektrischen Feld und beeinflusst es. Die elektrische Prüfung des Textilmaterials 9 basiert darauf, die Einflüsse des Textilmaterials 9 auf das elektrische Feld zu detektieren und daraus auf physikalische Eigenschaften des Textilmaterials 9 zu schliessen. Die kapazitive Prüfung von Textilmaterial ist aus dem Stand der Technik hinreichend bekannt. Im vorliegenden Ausführungsbeispiel dienen zwei Elektroden 142.1, 142.3 als Senderelektroden, die anderen zwei Elektroden 142.2, 142.4 als Empfängerelektroden.

Analog zur optischen Schnittstelle 55 und zum optischen Sender- und Empfängermodul 65 ist die Vorrichtung 1 gemäss Figur 6 mit einer elektrischen Schnittstelle 155 und einem elektrischen Anschlussteil 165 ausgestattet. Die elektrische Schnittstelle 155 kann als Steckverbindung ausgeführt sein, wie sie aus der Elektronik hinreichend bekannt und auf dem Markt erhältlich sind. Im elektrischen Anschlussteil 165 sind schematisch elektrische Bauteile 164 eingezeichnet, die z. B. Verstärker, Filter, Modulatoren oder Demodulatoren sein können. Das elektrische Anschlussteil 165 ist mittels einer oder mehrerer elektrischer Leitungen 171 mit der Elektronikeinheit 70 verbunden. Für das optische Sender- und Empfängermodul 65 und für das elektrische Anschlussteil 165 können mechanische Positioniermittel 53 vorgesehen sein, wie oben beschrieben.

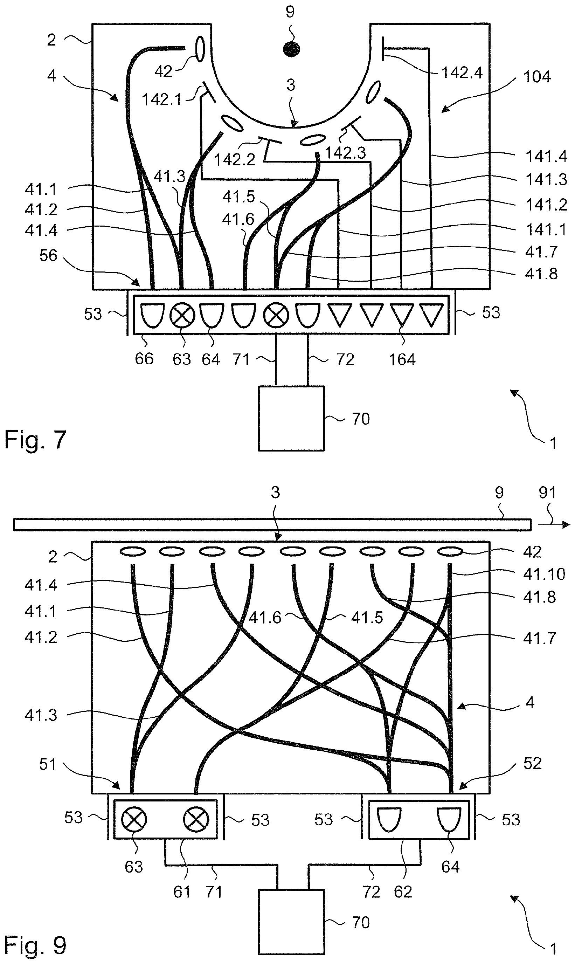

Die sechste Ausführungsform der erfindungsgemässen Vorrichtung 1, die in **Figur 7** dargestellt ist, entspricht weitgehend derjenigen von Figur 6. In der Ausführungsform von Figur 7 weist jedoch das Substrat 2 ausserhalb des Abtastbereichs 3 eine kombinierte optisch-elektrische Schnittstelle 56 auf Diese optisch-elektrische Schnittstelle 56 verbindet die optische Wellenleiterstruktur 4 und die elektrische Leiterstruktur 104 mit einem optisch-elektrischen Anschlussteil 66. Die optisch-elektrische Schnittstelle 56 weist mechanische Positioniermittel 53 zum Positionieren des optisch-elektrischen Anschlussteils 66 bezüglich des Substrates 2 auf Der Vorteil dieser Ausführungsform gegenüber derjenigen von Figur 6 besteht darin, dass bloss eine einzige Schnittstelle 56 und ein einziges entsprechendes Anschlussteil 66 benötigt werden. Wie bereits anlässlich der Figur 6 erläutert, können zwei Elektroden 142.1, 142.3 als Senderelektroden, die anderen zwei Elektroden 142.2, 142.4 als Empfängerelektroden dienen.

**Figur 8** zeigt eine siebte Ausführungsform der erfindungsgemässen Vorrichtung 1. Es handelt sich um eine kombinierte integriert-optisch und integriert-elektrische Vorrichtung 1, wie in Figur 6. Im Unterschied zur Ausführungsform von Figur 6 sind hier aber die Lichtquellen 63 und die Lichtempfänger 64 nicht in einem externen Sender- und Empfängermodul angebracht, sondern auf dem Substrat 2. Die Lichtquellen 63 und die Lichtempfänger 64 können als integriert-optische Bauteile auf dem Substrat 2 integriert sein oder als eigenständige Bauteile auf das Substrat 2 aufgebracht werden. Alternativ können sich solche Bauteile ausserhalb des Substrates 2 befinden, und das Licht kann mit entsprechenden Koppelelementen, die an sich bekannt sind, in die Beleuchtungswellenleiter eingekoppelt bzw. aus den Detektionswellenleitem ausgekoppelt werden.

Die zwei Lichtquellen 63 und die zwei Senderelektroden 142.1, 142.3 werden z. B. von je einem Signalgenerator 167 angesteuert. Die Signalgeneratoren 167 sind in Figur 8 als Mikroprozessoren dargestellt; dem Fachmann auf dem Gebiet der Elektronik sind jedoch viele andere Arten von Signalgeneratoren bekannt. Die Mikroprozessoren 167 sind vorzugsweise integrierte Schaltkreise in eigenen Gehäusen und werden bspw. mittels einer Steck- und/oder Lötverbindung auf das Substrat aufgebracht. Zwischen den Signalgeneratoren 167 und den Senderelektroden 142.1, 142.3, und möglicherweise auch den Lichtquellen 63, können elektronische Bauteile 167 wie Verstärker oder Filter vorgesehen sein. Möglichst nahe hinter den Lichtempfängern 64 bzw. den Empfängerelektroden 142.2, 142.4 sind jeweils Vorverarbeitungseinheiten 169 angebracht, welche die Aufgabe haben, die betreffenden elektrischen Ausgangssignale vorzuverarbeiten. Die Vorverarbeitung kann z. B. eine Vorverstärkung, eine Filterung und/oder eine Demodulation umfassen. Die derart vorverarbeiteten Signale werden einer elektrischen Schnittstelle 155, die sich vorzugsweise an einem Rand des Substrates 2 befindet, zugeführt. Die Schnittstelle 155 kann gleichzeitig zur Zuführung von elektrischen Signalen, bspw. Steuersignalen, und von elektrischer Energie in die elektrische Leiterstruktur 104 dienen. Sie kann als an sich bekannter elektrischer Mehrfachstecker ausgeführt sein.

**Figur 9** veranschaulicht, dass der Abtastbereich nicht, wie in den bisher diskutierten Ausführungsformen, halbrund zu sein braucht. In der achten Ausführungsform gemäss Figur 9 liegt eine Längsachse und Bewegungsrichtung 91 des Textilmaterials 9 in der Ebene des Substrates 2, aber ausserhalb des Substrates 2. Der Abtastbereich 3 fällt mit einem Teil einer Seite des rechteckigen Substrates 2 zusammen, ist gerade und parallel zur Längsachse 91 des Textilmaterials 9 angeordnet. Entlang des Abtastbereiches 3, vorzugsweise ebenfalls auf einer Gerade liegend, sind Sammellinsen 42 angeordnet. Sie befinden sich an Mündungen von Wellenleitern 41.1-41.8, die eine Wellenleiterstruktur 4 bilden. Im Ausführungsbeispiel von Figur 9 sind vier Beleuchtungswellenleiter 41.1, 41.3, 41.5, 41.7 und fünf Detektionswellenleiter 41.2, 41.4, 41.6, 41.8, 41.10 vorhanden. An einem anderen Rand des Substrates 2 sind ein Sendermodul 61 und ein Empfängermodul 62 angebracht. Der rechte der beiden im Empfängermodul 62 angebrachten Lichtempfänger 64 empfängt und addiert Licht von allen fünf Detektionswellenleitem 41.2, 41.4, 41.6, 41.8, 41.10, während der linke Lichtempfänger 64 nur Licht von jedem zweiten Detektionswellenleiter 41.2, 41.6, 41.10 empfängt und addiert. Eine derartige Addition von Licht, das an verschiedenen, vorzugsweise äquidistanten Stellen des Textilmaterials 9 reflektiert wurde, kann zusätzliche Information über das Textilmaterial 9 liefern. Abgesehen von der Ausführung des Abtastbereiches 3, von der Lage des Substrates 2 bezüglich der Längsachse 91 des Textilmaterials 9 und von der Wellenleiterstruktur 4 ist diese Ausführungsform analog zu derjenigen von Figur 2, und für einander entsprechende Elemente werden dieselben Bezugszeichen verwendet. Somit erübrigen sich an dieser Stelle weitere Erklärungen dazu.

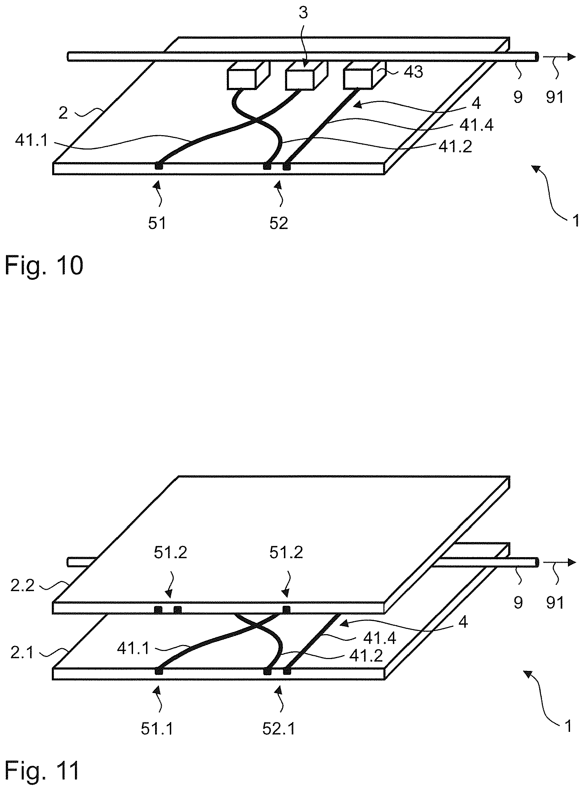

In der **Figur 10** ist eine neunte Ausführungsform der erfindungsgemässen Vorrichtung 1 dargestellt, in welcher die Längsachse 91 des Textilmaterials 9 parallel zur Ebene des Substrates 2 liegt, aber von dieser beabstandet ist. Somit wird das Textilmaterial 9 über dem Substrat 2 entlang seiner Längsrichtung 91 bewegt. Der Abtastbereich 3 liegt in oder über der Ebene des Substrates 2. Licht, das mittels eines Beleuchtungswellenleiters 41.1 von einer Einkoppelschnittstelle 51 zum Abtastbereich 3 geführt wird, wird im Abtastbereich 3 zum Textilmaterial 9 hin ausgekoppelt. Nach Wechselwirkung mit dem Textilmaterial 9, bspw. Reflexion und/oder Streuung an demselben, wird zumindest ein Teil dieses Lichtes in Detektionswellenleiter 41.2, 41.4 eingekoppelt und von diesen zu einer Auskoppelschnittstelle 52 geführt. Zum Aus- und Einkoppeln des Lichtes im Abtastbereich 3 werden in dieser Ausführungsform optische Koppelelemente 43 benötigt, die Licht aus der Ebene des Substrates 2 heraus bzw. von aussen in einen auf dem Substrat 2 integrierten Wellenleiter 41.2, 41.4 hinein koppeln können. Solche Koppelelemente 43 sind an sich bekannt und brauchen hier nicht näher diskutiert zu werden. Die Koppelelemente 43 können zusätzlich mit Sammellinsen und anderen optischen Bauteilen ausgerüstet sein. Sendermodul und Empfängermodul, Leitungen und Elektronikeinheit sind in Figur 10 der Einfachheit halber nicht eingezeichnet; sie können aber gleich oder ähnlich wie in den vorangehenden Figuren ausgeführt sein.

In der Ausführungsform von **Figur 11** läuft das Textilmaterial 9 zwischen zwei voneinander beabstandeten Substraten 2.1, 2.2 hindurch, wobei die Substratebenen parallel zueinander liegen und die Längsachse 91 des Textilmaterials 9 ebenfalls parallel zu den Substratebenen liegt. Die Substrate 2.1, 2.2 und die darauf integrierten Wellenleiterstrukturen sowie (in Figur 11 nicht sichtbare) Koppelelemente 43 können ähnlich ausgeführt sein wie das Substrat 2 in der Ausführungsform gemäss Figur 10. Der Vorteil der Ausführungsform gemäss Figur 11 besteht darin, dass auch am Textilmaterial 9 vorbei und/oder durch das Textilmaterial 9 hindurch transmittiertes Licht detektiert werden kann. Zu diesem Zweck wird einem Auskoppelelement auf dem ersten Substrat 2.1 ein Einkoppelelement auf dem zweiten Substrat 2.2 derart zugeordnet, dass das Einkoppelelement Licht des Auskoppelelementes empfängt, und umgekehrt. Solche Transmissionsanordnungen werden zur Bestimmung einer Querdimension und/oder der Haarigkeit des Textilmaterials 9 verwendet, während Reflexionsanordnungen gemäss Figur 10 zur Detektion von Fremdstoffen und/oder optischen Eigenschaften des Textilmaterials gebraucht werden. Beide Substrate 2.1, 2.2 sind mit Einkoppelschnittstellen 51.1, 51.2 und Auskoppelschnittstellen 52.1, 52.2 ausgestattet.

**Figur 12** zeigt eine Ausführungsform der erfindungsgemässen Vorrichtung 1 mit einem flexiblen oder biegsamen Substrat 2. Diese Eigenschaft wird hier ausgenützt, indem das Substrat 2 nicht eben, sondern zu einem Zylindermantel gebogen ist. Um der Vorrichtung 1 eine grössere mechanische Stabilität zu verleihen, ist es vorteilhaft, ein Stützelement 20 für das Substrat 2 vorzusehen. Im Ausführungsbeispiel von Figur 12 ist das Stützelement als ein zylindrisches Rohr 20 ausgebildet, an dessen Innenwand das gebogene Substrat 2 angebracht ist. Durch das Innere des Zylinders, vorzugsweise entlang einer Zylinderachse, läuft das Textilmaterial 9. Der Abtastbereich 3 verläuft entlang des Umfangs des Substrates 2 und kann sich nach Bedarf mehr oder weniger in axialer Richtung erstrecken. Das Textilmaterial 9 kann in dieser Ausführungsform entlang seines ganzen Umfangs optisch abgetastet werden. Auf dem Substrat 2, gegen das Textilmaterial 9 gerichtet, sind mehrere, bspw. sechs, Koppelemente 43 angebracht, wie sie bereits anlässlich der Figuren 10 und 11 beschreiben wurden. Unter den Koppelelementen 43 sind mindestens ein Auskoppelelement und mindestens ein Einkoppelelement vorhanden. Den Koppelelementen 43 sind optische Wellenleiter 41 zugeordnet, die nicht nur in Umfangrichtung, sondern auch in axialer Richtung verlaufen können und deshalb in der Querschnittansicht von Figur 12 nur teilweise sichtbar sind.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Insbesondere können die diskutierten Ausführungsformen beliebig miteinander kombiniert werden. Obwohl vielen der beiliegenden Zeichnungen aus ästhetischen Gründen achsensymmetrische Substratformen und Wellenleiterstrukturen gezeichnet sind, ist eine derartige Symmetrie für die vorliegende Erfindung nicht nötig. In der Praxis wird man möglicherweise, je nach Anwendung, asymmetrische Anordnungen bevorzugen. Auch ist die in den Zeichnungen beispielhaft verwendete Anzahl von Lichtquellen, Lichtempfängern, Wellenleitern, Wellenleiterenden, Linsen etc. keineswegs einschränkend zu verstehen.

### BEZUGSZEICHENLISTE

- 1: Vorrichtung

- 2: Substrat
- 20: Stützelement

- 3: Abtastbereich
- 31: Lichtstrahlen

- 4: optische Wellenleiterstruktur
- 41: optischer Wellenleiter
- 42: Sammellinse
- 43: optisches Koppelelement

- 51: Einkoppelschnittstelle
- 52: Auskoppelschnittstelle
- 53: mechanische Positioniermittel
- 55: optische Schnittstelle
- 56: optisch-elektrische Schnittstelle

- 61: Sendermodul
- 62: Empfängermodul
- 63: Lichtquelle
- 64: Lichtempfänger
- 65: Sender- und Empfängermodul
- 66: optisch-elektrisches Anschlussteil

- 70: Elektronikeinheit
- 71, 72: elektrische Leitungen

- 9: Textilmaterial
- 91: Längsachse und Bewegungsrichtung des Textilmaterials

- 104: elektrische Leiterstruktur

- 141: elektrische Leiterbahn
- 142: Elektrode

- 155: elektrische Schnittstelle

- 164: elektrisches Bauteil
- 165: elektrisches Anschlussteil
- 167: Signalgenerator

## Patentansprüche

1. Verwendung eines Substrats (2) mit mindestens einer darauf integrierten optischen Wellenleiterstruktur (4), die
monolithisch in oder auf dem Substrat (2) untergebracht ist,
originär auf dem Substrat hergestellt wurde,
untrennbar mit dem Substrat (2) verbunden ist und
eine Mehrzahl von transparenten dielektrischen Schichten mit unterschiedlichen Brechzahlen beinhaltet,
zur optischen Prüfung eines bewegten Textilmaterials (9), wobei auf dem Substrat (2) ein Abtastbereich (3) zum optischen Abtasten des bewegten Textilmaterials (9) vorgesehen ist.

2. Verwendung nach Anspruch 1, wobei auf dem Substrat (2) zusätzlich eine elektrische Leiterstruktur (104) zur elektrischen Prüfung des bewegten Textilmaterials (9) integriert ist.

3. Vorrichtung (1) zur optischen Prüfung eines bewegten Textilmaterials (9), mit einem Substrat (2), auf dem ein Abtastbereich (3) zum optischen Abtasten des Textilmaterials (9) vorgesehen ist,
**gekennzeichnet durch**
eine auf dem Substrat (2) integrierte optische Wellenleiterstruktur (4), die
monolithisch in oder auf dem Substrat (2) untergebracht ist,
originär auf dem Substrat hergestellt wurde,
untrennbar mit dem Substrat (2) verbunden ist,
eine Mehrzahl von transparenten dielektrischen Schichten mit unterschiedlichen Brechzahlen beinhaltet und
zumindest teilweise in den Abtastbereich (3) mündet.

4. Vorrichtung (1) nach Anspruch 3, wobei mindestens zwei optische Wellenleiter (41.1-41.10) der optischen Wellenleiterstruktur (4) in den Abtastbereich (3) münden.

5. Vorrichtung (1) nach Anspruch 4, wobei mindestens zwei Wellenleiter (41.1, 41.3, 41.5, 41.7) zur Leitung von Licht zum Abtastbereich (3) hin und mindestens zwei Wellenleiter (41.2, 41.4, 41.6, 41.8, 41.10) zur Leitung von Licht vom Abtastbereich (3) weg ausgebildet sind.

6. Vorrichtung (1) nach Anspruch 5, wobei die Mündungen der hinleitenden bzw. der wegleitenden Wellenleiter (41.1-41.10) abwechslungsweise nebeneinander angeordnet sind.

7. Vorrichtung (1) nach einem der Ansprüche 3-6, wobei mindestens eine Mündung eines Wellenleiters (41.1-41.10) in den Abtastbereich (3) mit einer Sammellinse (42) versehen ist.

8. Vorrichtung (1) nach einem der Ansprüche 3-7, wobei die optische Wellenleiterstruktur (4) mindestens eine Verzweigung mit mindestens zwei Ästen aufweist.

9. Vorrichtung (1) nach Anspruch 8, wobei mindestens zwei der Äste dem Abtastbereich (3) zugewandt sind.

10. Vorrichtung (1) nach Anspruch 8 oder 9, wobei mindestens zwei der Äste vom Abtastbereich (3) abgewandt sind.

11. Vorrichtung (1) nach einem der Ansprüche 3-10, wobei das Substrat (2) ausserhalb des Abtastbereichs (3) mindestens eine optische Schnittstelle (51, 52, 55, 56) zum Verbinden der mindestens einen optischen Wellenleiterstruktur (4) mit jeweils einem optischen Anschlussteil (61, 62, 65, 66) aufweist.

12. Vorrichtung (1) nach einem der Ansprüche 3-11, wobei auf dem Substrat (2) zusätzlich eine elektrische Leiterstruktur (104) integriert ist, die zumindest teilweise in den Abtastbereich (3) mündet.

13. Vorrichtung (1) nach Anspruch 12, wobei mindestens zwei elektrische Leiterbahnen (141.1-141.4) der elektrischen Leiterstruktur (104) in den Abtastbereich (3) münden.

14. Vorrichtung (1) nach Anspruch 12 oder 13, wobei mindestens eine Mündung einer elektrischen Leiterbahn (141.1-141.4) im Abtastbereich (3) mit einer Elektrode (142.1-142.4) versehen ist.

15. Vorrichtung (1) nach einem der Ansprüche 12-14, wobei das Substrat (2) ausserhalb des Abtastbereichs (3) mindestens eine elektrische Schnittstelle (155) zum Verbinden der mindestens einen elektrischen Leiterstruktur (104) mit jeweils einem elektrischen Anschlussteil (165) aufweist.

## Claims

1. The use of a substrate (2) with at least one optical waveguide structure (4) integrated thereon, which
is accommodated monolithically in or on the substrate (2),
was originally produced on the substrate,
is inseparably connected to the substrate (2) and
contains a plurality of transparent dielectric layers with different refractive indexes,
for the optical inspection of a moving textile material (9), wherein a scanning region (3) for optically scanning the textile material (9) is provided on the substrate (2).

2. The use according to claim 1, wherein an electrical conductor structure (104) for the electrical inspection of the moving textile material (9) is additionally integrated on the substrate (2).

3. A device (1) for the optical inspection of a moving textile material (9), with a substrate (2) on which a scanning region (3) for optically scanning the textile material (9) is provided,
**characterized by**
an optical waveguide structure (4) which is integrated on the substrate (2) and which
is accommodated monolithically in or on the substrate (2),
was originally produced on the substrate,
is inseparably connected to the substrate (2),
contains a plurality of transparent dielectric layers with different refractive indexes and
opens at least partly into the scanning region (3).

4. A device (1) according to claim 3, wherein at least two optical waveguides (41.1 to 41.10) of the optical waveguide structure (4) open into the scanning region (3).

5. A device (1) according to claim 4, wherein at least two waveguides (41.1, 41.3, 41.5, 41.7) are arranged for guiding light towards the scanning region (3), and at least two waveguides (41.2, 41.4, 41.6, 41.8, 41.10) are arranged for guiding light away from the scanning region (3).

6. A device (1) according to claim 5, wherein the orifices of the waveguides (41.1 to 4.10) guiding towards and away are arranged adjacent to each other in an alternating fashion.

7. A device (1) according to one of the claims 3 to 6, wherein at least one orifice of a waveguide (41.1 to 4.10) into the scanning region (3) is provided with a focusing lens (42).

8. A device (1) according to one of the claims 3 to 7, wherein the optical waveguide structure (4) comprises at least one junction with at least two branches.

9. A device (1) according to claim 8, wherein at least two of the branches face the scanning region (3).

10. A device (1) according to claim 8 or 9, wherein at least two of the branches face away from the scanning region (3).

11. A device (1) according to one of the claims 3 to 10, wherein the substrate (2) outside of the scanning region (3) comprises at least one optical interface (51, 52, 55, 56) for connecting the at least one optical waveguide structure (4) to a respective optical connecting part (61, 62, 65, 66).

12. A device (1) according to one of the claims 3 to 11, wherein an electrical conductor structure (104) is additionally integrated on the substrate (2), which structure opens at least partly into the scanning region (3).

13. A device (1) according to claim 12, wherein at least two electrical conductor paths (141.1 to 141.4) of the electrical conductor structure (104) open into the scanning region (3).

14. A device (1) according to claim 12 or 13, wherein at least one orifice of an electrical conductor path (141.1 to 141.4) is provided with an electrode (142.1 to 142.4) in the scanning region (3).

15. A device (1) according to one of the claims 12 to 14, wherein the substrate (2) comprises at least one electrical interface (155) for connecting the at least one electrical conductor structure (104) to a respective electrical connecting part (165) outside of the scanning region (3).

## Revendications

1. Utilisation d'un substrat (2) sur lequel est intégrée au moins une structure de guides d'ondes optiques (4) qui
est logée de façon monolithique dans ou sur le substrat (2),
a été réalisée originairement sur le substrat,
est reliée de manière inséparable au substrat (2) et
comprend une pluralité de couches diélectriques transparentes ayant des indices de réfraction différents,
pour le contrôle optique d'un matériau textile en mouvement (9), une zone de balayage (3) pour le balayage optique du matériau textile en mouvement (9) étant prévue sur le substrat (2).

2. Utilisation selon la revendication 1, dans laquelle une structure de conducteurs électriques (104) pour le contrôle électrique du matériau textile en mouvement (9) est en outre intégrée sur le substrat (2).

3. Dispositif (1) pour le contrôle optique d'une matière textile en mouvement (9), comportant un substrat (2) sur lequel est prévue une zone de balayage (3) pour le balayage optique du matériau textile (9),
**caractérisé par**
une structure de guides d'ondes optiques (4) intégrée sur le substrat (2) qui
est logée de façon monolithique dans ou sur le substrat (2),
a été réalisée originairement sur le substrat,
est reliée de manière inséparable au substrat (2) et
comprend une pluralité de couches diélectriques transparentes ayant des indices de réfraction différents et
débouche au moins partiellement dans la zone de balayage (3).

4. Dispositif (1) selon la revendication 3, dans lequel au moins deux guides d'ondes optiques (41.1-41.10) de la structure de guides d'ondes optiques (4) débouchent dans la zone de balayage (3).

5. Dispositif (1) selon la revendication 4, dans lequel au moins deux guides d'ondes (41.1, 41.3, 41.5, 41.7) sont formés pour conduire la lumière vers la zone de balayage (3) et au moins deux guides d'ondes (41.2, 41.4, 41.6, 41.8, 41.10) sont formés pour conduire la lumière à partir de la zone de balayage (3).

6. Dispositif (1) selon la revendication 5, dans lequel les débouchés des guides d'ondes d'amenée (41.1-41.10) et des guides d'ondes de départ (41.1-41.10) sont disposés alternativement les uns à côté des autres.

7. Dispositif (1) selon l'une quelconque des revendications 3 à 6, dans lequel au moins un débouché d'un guide d'ondes (41.1-41.10) dans la zone de balayage (3) est pourvu d'une lentille convergente (42).

8. Dispositif (1) selon l'une des revendications 3 à 7, dans lequel la structure de guides d'ondes optiques (4) présente au moins un embranchement à au moins deux branches.

9. Dispositif (1) selon la revendication 8, dans lequel au moins deux des branches sont tournées vers la zone de balayage (3).

10. Dispositif (1) selon la revendication 8 ou 9, dans lequel au moins deux des branches sont tournées à l'opposé de la zone de balayage (3).

11. Dispositif (1) selon l'une des revendications 3 à 10, dans lequel le substrat (2) présente, en dehors de la zone de balayage (3), au moins une interface optique (51, 52, 55, 56) pour relier ladite au moins une structure de guides d'ondes optiques (4) à une partie de connexion optique respective (61, 62, 65, 66).

12. Dispositif (1) selon l'une des revendications 3 à 11, dans lequel une structure de conducteurs électriques (104) est en outre intégrée sur le substrat (2), laquelle débouche au moins partiellement dans la zone de balayage (3).

13. Dispositif (1) selon la revendication 12, dans lequel au moins deux pistes conductrices électriques (141.1-141.4) de la structure de conducteurs électriques (104) débouchent dans la zone de balayage (3).

14. Dispositif (1) selon la revendication 12 ou 13, dans lequel au moins un débouché d'une piste conductrice électrique (141.1-141.4) est pourvu d'une électrode (142.1-142.4) dans la zone de balayage (3).

15. Dispositif (1) selon l'une des revendications 12 à 14, dans lequel le substrat (2) présente, en dehors de la zone de balayage (3), au moins une interface électrique (155) pour relier ladite au moins une structure de conducteurs électriques (104) à une partie de connexion électrique respective (165).
